# EUROPÄISCHE PATENTSCHRIFT

(11) **EP 3 888 576 B1**
(45) Veröffentlichungstag und Bekanntmachung des Hinweises auf die Patenterteilung: **22.03.2023**
(21) Anmeldenummer: 20167055.1
(22) Anmeldetag: 31.03.2020
(51) Int. Cl.: A61B 17/72, A61B 17/56

(54) **MARKNAGEL ZUR APPLIKATION VON PHARMAZEUTISCHEN FLUIDEN**
INTRAMEDULLARY NAIL FOR APPLICATION OF PHARMACEUTICAL FLUIDS
CLOU MÉDULLAIRE DESTINÉ À L'APPLICATION DE FLUIDES PHARMACEUTIQUES

(43) Veröffentlichungstag der Anmeldung: 06.10.2021
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 2 399 533
- CN-Y- 2 857 862
- CN-Y- 201 370 624
- US-A1- 2019 175 232

## Beschreibung

Die Erfindung betrifft einen Marknagel zur Applikation eines pharmazeutischen Fluids, umfassend ein axial im Marknagel verlaufendes fluidleitendes erstes Leitungsmittel, welches mit einem ersten Reservoir für das pharmazeutische Fluid verbindbar ist und mindestens eine Durchführung, welche das erste Leitungsmittel fluidleitend mit einer Außenfläche des Marknagels verbindet.

### Hintergrund der Erfindung

Zur chirurgischen Versorgung von offenen, als auch von geschlossenen, Knochenfrakturen, insbesondere zur Behandlung bei Knochenfrakturen von großen Röhrenknochen, hat sich seit Jahrzehnten der Einsatz von Marknägeln bewährt. Dazu wird zur Reposition und Stabilisierung des frakturierten Knochens ein Marknagel in den Knochenkanal eingeführt und fixiert, welcher zumindest bis zur Heilung der Knochenfraktur innerhalb des Knochenkanals verbleibt.

Durch Knochenfrakturen, Insbesondere durch offene Knochenfrakturen, kann es zu Kontaminationen des Knochengewebes mit Mikroorganismen kommen. Die so ausgelösten Infektionen, wie beispielsweise eine Osteitis, insbesondere eine Osteomyelitis, stellen sehr schwerwiegende Erkrankungen dar.

Zur Behandlung der mikrobiellen Infektionen ist nach einem chirurgischen Debridement des infizierten Knochengewebes eine lokale Gabe von Antibiotika üblich.

In der US 8,609,003 B2 und der US 5,618,286 A werden Marknägel beschrieben, welche mit PMMA-Knochenzement umhüllt sind, der ein oder mehrere Antibiotika enthält. Nachteilig gestaltet sich dabei, dass die im PMMA-Knochenzement inkorporierten Antibiotika nur innerhalb der ersten Tage nach der Implantation des Marknagels eine ausreichend hohe Konzentration an Antibiotika freisetzen können, was für eine abschließende Bekämpfung der Infektion zu kurz sein kann. Weiterhin kann die Antibiotikazusammensetzung nach erfolgter Implantation nicht variiert werden, was insbesondere im Falle einer Unwirksamkeit der im PMMA-Knochenzement enthaltenen Antibiotika nachteilig ist.

Eine Weiterentwicklung stellen daher Marknägel mit perforiertem Marknagelkörper dar, mittels derer nach erfolgter Implantation über ein Leitungsmittel verschiedene pharmazeutische Fluide, wie beispielsweise antiseptische Wirkstofflösungen, lokal am infizierten Knochengewebe über Zuleitung von außen appliziert werden kann. Solche Marknägel werden beispielsweise in der US 5,681,289 A, der CN 2857862 Y und der CN 201370624 Y beschrieben. Dabei kann eine antiseptische Wirkstofflösung durch Durchführungen in den entsprechenden Marknägeln austreten und das infizierte Knochengewebe erreichen.

Ein Nachteil der beschriebenen Marknägel besteht in einer ungewollten Anreicherung des im pharmazeutischen Fluids enthaltenen Wirkstoffs im Patientenkörper, welche durch den beim Einpressen des Fluids in den Knochenkanal benötigten, hohen Applikationsdruck verursacht wird. Bei einer Reihe von Antibiotika, wie beispielsweise Aminoglykosid-Antibiotika, können hohe systemische Antibiotika-Konzentrationen Schädigungen des Patienten, wie beispielsweise Nierenschäden und Schäden am Hörnerv, verursachen. Weiterhin wird durch den durch die Applikation hervorgerufenen Druckaufbau das Risiko von Embolien erhöht. Zudem kann zu einem unkontrolliertem Rückfließen des pharmazeutischen Fluids durch den zuvor zur Einleitung genutzten Zugang, und damit zur Kontamination des Spülsystems, insbesondere des Marknagels, kommen.

EP2399533 offenbart einen Marknagel nach dem Oberbegriff des Anspruchs 1.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, Marknägel bereitzustellen, welche in der Lage sind den Innenraum von Knochen, insbesondere von Röhrenknochen, mit pharmazeutischen Fluiden beliebiger Zusammensetzung, insbesondere mit antiseptischen Wirkstofflösungen, wie beispielsweise antibiotikahaltigen Wirkstofflösungen, zu spülen. Dabei soll das pharmazeutische Fluid von einem außerhalb des Patientenkörpers angeordneten Reservoir dem Marknagel zuführbar sein und gleichzeitig Nebenwirkungen, wie beispielsweise die Anreicherung des zugeführten Wirkstoffs im Patientenkörper und die Ausbildung von Embolien, insbesondere von Fettembolien, vermieden werden. Das Ziel ist zudem, die Wirkstofflösung zuverlässig über den Innenraum der Knochen zu verteilen und gleichzeitig eine hohe mechanische Stabilität der Marknägel und eine sichere Fixierung derselben im Knochen zu gewährleisten. Zudem sollen die Marknägel ein sicheres und kontrollierbares Spülen eines Innenraums eines Knochens ermöglichen, ohne dass es dabei zu einer Kontamination oder einer Verstopfung der Marknägel, insbesondere durch Eindringen von Knochengewebe in die Marknägel, kommt.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft einen Marknagel zur Applikation eines pharmazeutischen Fluids, umfassend
ein axial im Marknagel verlaufendes fluidleitendes erstes Leitungsmittel, welches mit einem ersten Reservoir für das pharmazeutische Fluid verbindbar ist und
mindestens eine Durchführung, welche das erste Leitungsmittel fluidleitend mit einer Außenfläche des Marknagels verbindet,
dadurch gekennzeichnet, dass
ein fluidleitendes zweites Leitungsmittel die Außenfläche derart mit einem zweiten Reservoir verbindet, um das pharmazeutische Fluid zwischen dem ersten Reservoir und dem zweiten Reservoir zu fördern.

Ein Marknagel stellt eine intramedulläre Schienung bei der Behandlung einer Knochenfraktur insbesondere eines Röhrenknochens dar, welche den Knochen bis zur Heilung der Knochenfraktur stabilisiert. In der Praxis kommen überwiegend aufgebohrte und nicht-aufgebohrte Marknägel zum Einsatz, wobei sich die Begrifflichkeiten "aufgebohrt" und "nichtaufgebohrt" auf die Implantationsmethode der Marknägel bezieht. Bei aufgebohrten Marknägeln wird der gesamte Markraum des Röhrenknochens aufgebohrt und anschließend der Marknagel in den aufgebohrten Knochenkanal eingeschlagen. Eine mechanische Verriegelung zur Fixierung des Marknagels innerhalb des Knochenkanals ist nicht obligat.

Nicht-aufgebohrte Marknägel sind massiver, aber dünner als aufgebohrte Marknägel, wobei eine Fixierung mit Verriegelungsschrauben in der Regel geboten ist. Die erfindungsgemäße Marknagel kann sowohl als aufgebohrter Marknagel, als auch als nicht-aufgebohrter Marknagel verwendet werden, wobei die Verwendung als aufgebohrter Marknagel bevorzugt ist.

Der erfindungsgemäße Marknagel dient zur Applikation eines pharmazeutischen Fluids zur Behandlung infizierten Knochengewebes, insbesondere zur Applikation innerhalb des Knochenkanals des Knochens, über einen Zeitraum von mehreren Tagen bis Wochen. Ein pharmazeutisches Fluid enthält wenigstens einen pharmazeutischen Wirkstoff. Beispielsweise handelt es sich bei dem pharmazeutischen Fluid um wässrige oder nicht-wässrige Lösungen oder Suspensionen von pharmazeutischen Wirkstoffen.

In einer Ausgestaltungsform handelts es sich bei dem pharmazeutisches Fluid um Lösungen, welche wenigstens ein Antibiotikum, wenigstens ein Chemotherapeutikum und/oder wenigstens ein Antimykotikum enthalten. In einer weiteren Ausgestaltungsform enthalten die pharmazeutischen Fluide wenigstens einen desinfizierenden Bestandteil.

Weiterhin umfassen pharmazeutische Fluide auch Gase, Gasgemische und Lösungen von Gasen in Flüssigkeiten, wie beispielsweise Wasser.

Der Marknagel weist ein erstes Leitungsmittel und ein zweites Leitungsmittel auf. Unter einem Leitungsmittel werden alle Elemente verstanden, welche zwei Strukturen fluidleitend miteinander verbinden können. Beispiele für solche Elemente sind rohrartige, schlauchartige oder kanalartige Verbindungen.

Um eine für die Applikation des pharmazeutischen Fluids ausreichend hohe Durchflussrate zu gewährleisten, kann das erste Leitungsmittel und/oder das zweite Leitungsmittel beispielsweise einen Innenquerschnitt von 0,5 mm² bis 5 mm², bevorzugt von 1 mm² bis 4 mm² aufweisen. Um ein Fördern von Feststoffen, und damit ein Risiko einer Verstopfung des ersten Leitungsmittels und/oder des zweiten Leitungsmittel zu verhindern kann das erste Leitungsmittel und/oder das zweite Leitungsmittel jeweils ein Sieb aufweisen oder aus einem Sieb bestehen.

Das erste Leitungsmittel verläuft zumindest partiell axial innerhalb des Marknagels. Der axiale Verlauf des ersten Leitungsmittel innerhalb des Marknagels kann auf unterschiedliche Weisen ausgestaltet sein. Aufgrund der leichten Fertigungsweise verläuft das erste Leitungsmittel bevorzugt zumindest partiell parallel zur Längsachse des Marknagels.

Die Wegstrecke, die das erste Leitungsmittel innerhalb des Marknagels axial verläuft, kann unterschiedliche lang ausgestaltet sein. Beispielsweise kann das erste Leitungsmittel durch 1-100% einer Gesamtlänge des Marknagels axial verlaufen. Das erste Leitungsmittel kann beispielsweise mindestens 1 %, 10 %, 70 %, 80 % der Gesamtlänge des Marknagels axial durchlaufen. Als Obergrenze kann das erste Leitungsmittel maximal 100 %, 95 %, 90 % oder 85 % der Gesamtlänge des Marknagels axial durchlaufen.

Der Marknagel ist über das erste Leitungsmittel mit einem ersten Reservoir und über das zweite Leitungsmittel mit einem zweiten Reservoir für ein pharmazeutisches Fluid fluidleitend verbindbar. Unter einem Reservoir werden alle Behältnisse verstanden, welche dazu geeignet sind, das pharmazeutische Fluid bereitzustellen und/oder aufzunehmen. Beispiele für Reservoire umfassen Beutel, Spritzen, Kolben, Ballons, Kanister und Ampullen, wobei Beutel, Ballons und Spritzen bevorzugt sind. Als ein Reservoir zur Aufnahme des pharmazeutischen Fluids kann es sich zudem darüber hinaus um einen Beutel, ein Gefäß oder einen andersartigen Auffangbehälter handeln, welcher nicht strukturell mit dem ersten Leitungsmittel oder dem zweiten Leitungsmittel verbunden sein muss, aber dazu geeignet ist, das aus dem ersten Leitungsmittel oder dem zweiten Leitungsmittel austretende pharmazeutische Fluid aufzufangen. In einer Ausgestaltungsform kann das das pharmazeutische Fluid aufnehmende Reservoir auch ein Raum oder ein Boden eines Raums sein, in den das aus dem ersten Leitungsmittel oder dem zweiten Leitungsmittel austretende pharmazeutische Fluid ausgeleitet wird oder auf den das, in diesem Fall flüssige pharmazeutische Fluid, fließt oder tropft.

Das erste Leitungsmittel ist über mindestens eine Durchführung mit einer Außenfläche des Marknagels fluidleitend verbunden, wobei die Außenfläche die Fläche des Marknagels darstellt, welche in physischem Kontakt mit dem Knochengewebe des Patienten steht. In einer Ausgestaltungsform ist die mindestens eine Durchführung als mindestens eine Perforation im Marknagel ausgestaltet, welche das erste Leitungsmittel mit der Außenfläche fluidleitend verbindet. Um eine für die Applikation des pharmazeutischen Fluids ausreichend hohe Durchflussrate durch die mindestens eine Durchführung zu gewährleisten, kann die Durchführung einen Innenquerschnitt von 0,5 mm² bis 5 mm², bevorzugt von 1 mm² bis 4 mm² von aufweisen. Um ein Fördern von Feststoffen, und damit ein Risiko einer Verstopfung der Durchführung zu verhindern, kann die mindestens eine Durchführung ein Sieb aufweisen, aus einem Sieb bestehen oder einen Verschluss, wie beispielsweise eine Klappe, aufweisen, welcher sich erst beim Fördern des pharmazeutischen Fluids durch die mindestens eine Durchführung öffnet, ansonsten aber verschlossen bleibt und somit ein Einwachsen von Gewebe, und damit einem Verstopfen der mindestens einen Durchführung, verhindert.

Das erste Reservoir ist über das erste Leitungsmittel, die mindestens eine Durchführung, die Außenfläche des Marknagels und das zweite Leitungsmittel fluidleitend mit dem zweiten Reservoir verbindbar, um das pharmazeutische Fluid zwischen dem ersten Reservoir und dem zweiten Reservoir zu fördern, so dass das pharmazeutische Fluid an das den Marknagel umgebende infizierte Knochengewebe applizierbar ist.

In einer Ausgestaltungsform ist das pharmazeutische Fluid aus dem ersten Reservoir über das erste Leitungsmittel, die mindestens eine Durchführung, die Außenfläche des Marknagels und das zweite Leitungsmittel in das zweite Reservoir förderbar. In einer weiteren Ausgestaltungsform ist das pharmazeutische Fluid aus dem zweiten Reservoir über das zweite Leitungsmittel, die Außenfläche des Marknagels, die mindestens eine Durchführung und das erste Leitungsmittel in das erste Reservoir förderbar.

In beiden Ausgestaltungsformen kommt das pharmazeutischen Fluid über die Außenfläche des Marknagels mit dem Knochengewebe des Patienten in Kontakt und erlaubt somit eine Applikation des in dem pharmazeutischen Fluids enthaltenen Wirkstoff zur Bekämpfung von Infektionen im Bereich der Knochenfraktur. Gleichzeitig verbleibt das pharmazeutische Fluid nicht im Bereich des infizierten Knochengewebes, sondern kann in das erste Reservoir oder das zweite Reservoir abgeführt werden. Somit erzeugt ein fortgeführtes Zuführen des pharmazeutischen Fluids keinen Druckaufbau durch im Knochenkanal verbleibendes Fluid, wodurch ein unerwünschtes erhöhtes Einbringen des Wirkstoffs in den Blutkreislauf des Patienten unterbunden wird. Dies verhindert ein Anreichern des Wirkstoffs im Patienten und verringert zudem das Risiko von Embolien.

Eine Ausgestaltungsform des Marknagels ist dadurch gekennzeichnet, dass das erste Leitungsmittel einen ersten Anschluss zur fluidleitenden Verbindung mit dem ersten Reservoir und das zweite Leitungsmittel einen zweiten Anschluss zur fluidleitenden Verbindung mit dem zweiten Reservoir umfasst, wobei der erste Anschluss und der zweite Anschluss an einem ersten Ende des Marknagels angeordnet sind.

Unter einem Anschluss ist eine Struktureinheit zu verstehen, welche ein fluidleitendes Verbinden von Leitungsmittel und Reservoir erlaubt. In einer Ausgestaltungsform ist der Anschluss als Tülle ausgeformt, über die das Leitungsmittel mittels Schlauchs mit dem Reservoir fluidleitend verbindbar ist. In einer weiteren Ausgestaltungsform ist der Anschluss als Gewinde ausgeformt, welches über ein entsprechendes Gegenstück fluidleitend mit dem Reservoir verbindbar ist. In einer weiteren Ausgestaltungsform formt der Anschluss mit dem Reservoir eine Flanschverbindung. In einer weiteren Ausgestaltungsform formt der Anschluss über eine Schlauchkupplung mit dem Reservoir eine fluidleitende Verbindung, wobei der Anschluss die Kupplung oder den Nippel der Schlauchkupplung aufweisen kann.

Der erste Anschluss und der zweite Anschluss sind gemeinsam an einem ersten Ende des Marknagels angeordnet, so dass das Zu- und Abführen des pharmazeutischen Fluids in beziehungsweise aus dem Knochenkanal am gleichen Ende des Knochens vonstattengehen kann. Vorzugsweise befindet sich der erste Anschluss und der zweite Anschluss an dem Ende des Marknagels, welches der für die Implantation des Marknagels vorgesehenen Öffnung des Knochens zugewandt ist. In einer bevorzugten Ausgestaltungsform bilden der erste Anschluss und der zweite Anschluss ein gemeinsames Strukturelement, wie beispielsweise eine gemeinsame Tülle, aus, welches die Verwendung eines Doppelschlauchsystems zum Zu- und Abführen des pharmazeutischen Fluids in beziehungsweise aus dem Marknagel erlaubt.

Eine Ausgestaltungsform des Marknagels ist dadurch gekennzeichnet, dass die mindestens eine Durchführung an einem dem ersten Ende entgegengesetzten zweitem Ende des Marknagels angeordnet ist. Somit erstreckt sich das erste Leitungsmittel vom ersten Ende des Marknagels, wo sich der erste Anschluss befindet, bis zum zweiten Ende des Marknagels, an dem die Durchführung das erste Leitungsmittel mit der Außenfläche des Marknagels fluidleitend verbindet. Vorzugsweise erstreckt sich das erste Leitungsmittel axial über 70-100 %, weiter bevorzugt über 80-100 %, noch weiter bevorzugt über 85-95 % der Gesamtlänge des Marknagels.

In einer bevorzugten Ausgestaltungsform ist das zweite Leitungsmittel derart ausgestaltet, beispielsweise als zumindest ein Kanal in den Marknagels hinein, dass ein Ableiten des pharmazeutischen Fluids von der Außenfläche des Marknagels am ersten Ende des Marknagels erfolgt. Bevorzugt wird das pharmazeutische Fluid zumindest über 60-100 %, bevorzugt 70-100 %, weiter bevorzugt zwischen 80-95 % der Gesamtlänge des Marknagel entlang der Außenfläche, beginnend mit der zumindest einen Durchführung, geleitet, bevor es mittels der ableitenden Ausgestaltung des zweiten Leitungsmittels von der Außenfläche gefördert wird. Dies sorgt dafür, dass das pharmazeutische Fluid einen Großteil der Gesamtlänge des Marknagel an der Außenfläche entlanggeführt wird, während ein Fördern zwischen dem ersten Reservoir und dem zweiten Reservoir durchgeführt wird. Damit erlaubt der Marknagel eine großflächige Behandlung infizierten Knochengewebes.

Das pharmazeutische Fluid kann auf unterschiedliche Weisen entlang der Außenfläche des Marknagels geführt werden.

Eine Ausgestaltungsform des Marknagel ist dadurch gekennzeichnet, dass das zweite Leitungsmittel zumindest partiell als mindestens eine sich axial erstreckende Nut in der Außenfläche ausgebildet ist. Durch die mindestens eine Nut kann das pharmazeutische Fluid sicher, in größeren Mengen und ohne das Risikos einer Verstopfung gefördert werden, wobei eine Benetzung der Außenfläche entlang der Nut eine wirksame Bekämpfung einer Infektion sicherstellt. Somit kommt es nicht zu einem Druckaufbau durch fortgeführtes Fördern des pharmazeutischen Fluids zwischen dem ersten Reservoir und dem zweiten Reservoir, was ein ungewolltes Anreichern des im pharmazeutischen Fluids enthaltenen Wirkstoffs unterbindet und das Risiko von Embolien vermindert. Bevorzugt ist das zweite Leitungsmittel als mehrere, beispielsweise als zwei bis zehn, axial verlaufende Nuten ausgebildet, welche radial über die Außenfläche des Marknagels verteilt sind.

Dies erlaubt eine großflächige Applikation des pharmazeutischen Fluids im gesamten Bereich des Marknagels bei gleichzeitig vermindertem Risiko eines ungewollten Druckaufbaus beim Fördern.

Die zumindest eine sich axial erstreckende Nut kann unterschiedlich ausgestaltet sein. In einer Ausgestaltungsform verläuft die zumindest eine sich axial erstreckende Nut in wellenförmigen Auslenkungen entlang der Außenfläche. In einer weiteren Ausgestaltungsform verläuft die mindestens eine Nut schraubenförmig entlang der Außenfläche. Aufgrund der leichteren Fertigung verläuft die zumindest eine sich axial erstreckende Nut im Wesentlichen parallel zur Längsachse des Marknagels.

Die zumindest eine Nut kann sich unterschiedlich lang entlang der Längsachse des Marknagels erstrecken. Beispielsweise kann sich die zumindest eine Nut über 5-100 %, bevorzugt über 50-100 %, weiter bevorzugt über 70-95 % der Gesamtlänge des Marknagels entlang der Außenfläche erstrecken.

Die mindestens eine Durchführung und das zweite Leitungsmittel können auf unterschiedliche Weise fluidleitend miteinander verbunden sein. In einer Ausgestaltungsform sind die mindestens eine Durchführung und das zweite Leitungsmittel, insbesondere in Form zumindest einer sich axial in der Außenfläche erstreckende Nut, nicht direkt, sondern nur über die Außenfläche des Marknagel miteinander fluidleitend verbunden.

Eine Ausgestaltungsform des Marknagels ist dadurch gekennzeichnet, dass die mindestens eine sich axial erstreckende Nut direkt fluidleitend mit der mindestens eine Durchführung verbunden ist, ohne dass dabei das pharmazeutische Fluid über die Außenfläche des Marknagels gefördert werden muss. Damit wird das Risiko eines ungewollten Druckaufbaus beim Fördern des pharmazeutischen Fluids zwischen dem ersten Reservoir und dem zweiten Reservoir weiter gesenkt.

Eine Ausgestaltungsform des Marknagels ist dadurch gekennzeichnet, dass das zweite Leitungsmittel als zwei bis sechs, insbesondere als vier, sich axial erstreckende Nuten in der Außenfläche ausgebildet ist und der Marknagel die gleiche Anzahl an Durchführungen aufweist, wobei jeweils eine Nut mit jeweils einer Durchführung direkt fluidleitend miteinander verbunden ist.

In einer Ausgestaltungsform erlaubt der Marknagel ein Fördern des pharmazeutischen Fluids sowohl aus dem ersten Reservoir in das zweite Reservoir als auch aus dem zweiten Reservoir in das erste Reservoir.

Um ein Fördern des pharmazeutischen Fluids in ausschließlich eine Flussrichtung, also aus dem ersten Reservoir in das zweite Reservoir oder aus dem zweiten Reservoir in das erste Reservoir, zuzulassen, ist eine Ausgestaltungsform des Marknagels dadurch gekennzeichnet, dass das erste Leitungsmittel ein erstes Rückschlagventil aufweist. Auf diese Weise ist kontrollierbar, welche Teile des Marknagels mit pharmazeutischem Fluid, welches bereits mit Knochengewebe in Kontakt gekommen ist, kontaminiert werden. Zudem erlaubt dies die Flussrichtung des pharmazeutischen Fluids durch den Marknagel zu wählen, welche das geringere Risiko einer Verstopfung mit sich bringt.

Eine Ausgestaltungsform des Marknagels ist dadurch gekennzeichnet, dass das erste Rückschlagventil in Richtung der mindestens einen Durchführung fluiddurchlässig und in Richtung des ersten Anschlusses fluidundurchlässig ausgestaltet ist. Dadurch erlaubt der Marknagel ausschließlich ein Fördern des pharmazeutischen Fluids aus dem ersten Reservoir in das zweite Reservoir. Ein Vorteil dieser Ausgestaltungsform ist, dass das nur unbenutztes pharmazeutisches Fluid durch das zumindest partiell axial im Marknagel verlaufende erste Leitungsmittel förderbar ist und eine Kontamination und/oder Verstopfung des ersten Leitungsmittel mit Knochengewebe, welches sich nach dem Kontakt mit Knochengewebe im pharmazeutischen Fluid ansammeln kann, ausgeschlossen wird.

Da in einer bevorzugten Ausgestaltungsform des Marknagels das erste Leitungsmittel den Marknagel durch den ersten Anschluss am ersten Ende zuführbar ist und am zweiten Ende durch die zumindest eine Durchführung wieder verlässt und gleichzeitig das zweite Leitungsmittel das pharmazeutische Fluid sowohl am ersten Ende von der Außenfläche des Marknagels abführt, als auch den Marknagel durch den zweiten Anschluss am ersten Ende wieder verlässt, wird durch das erste Rückschlagventil das länger im Marknagelinneren verlaufende der beiden Leitungsmittel vor Kontamination und/oder Verstopfung durch Knochengewebe, insbesondere durch Knochenmark, geschützt.

Das erste Rückschlagventil kann unterschiedliche Bauformen aufweisen. In einer Ausgestaltungsform umfasst das erste Rückschlagventil eine Rückschlagklappe, welche ein Fördern des pharmazeutischen Fluids durch das erste Leitungsmittel nur in eine Flussrichtung erlaubt und in die entgegengesetzte Flussrichtung das erste Leitungsmittel fluidleitend verschließt.

Ein Ausgestaltungsform des Marknagels ist dadurch gekennzeichnet, dass das erste Rückschlagventil ein erstes Rückstellelement aufweist, welche das erste Rückschlagventil in eine Richtung fluidleitend verschließt und in die entgegengesetzte Richtung ein Fördern des pharmazeutischen Fluids durch das erste Rückschlagventil erlaubt. Aufgrund der einfachen Bauweise und der sicheren Verwendbarkeit umfasst das Rückstellelement bevorzugt eine Feder, insbesondere eine Spiralfeder, beispielsweise aus einem Kunststoff oder aus einem Metall, oder besteht das erste Rückstellelement aus einer Feder, insbesondere einer Spiralfeder, beispielsweise aus einem Kunststoff oder aus einem Metall.

In einer Ausgestaltungsform ist das erste Rückschlagventil als Kugelrückschlagventil ausgebildet. Aufgrund der hohen strukturellen Robustheit ist in einer weiteren, bevorzugten, Ausgestaltungsform das erste Rückschlagventil als Tellerrückschlagventil ausgebildet.

Das erste Rückstellelement kann an unterschiedlichen Stellen innerhalb des ersten Leitungsmittels angeordnet sein, um ein Fördern des pharmazeutischen Fluids in nur eine Flussrichtung zu erlauben.

Um eine Kontamination und/oder eine Verstopfung des ersten Leitungsmittels möglichst effektiv zu verhindern, ist eine bevorzugte Ausgestaltungsform des Marknagels dadurch gekennzeichnet, das erste Rückstellelement am zweiten Ende des Marknagels angeordnet ist. Weiter bevorzugt ist das erste Rückstellelement am weitest axial vom ersten Ende des Marknagels entfernten Punkt des ersten Leitungsmittel angeordnet, insbesondere am Übergang vom ersten Leitungsmittel in die mindestens eine Durchführung.

Um ein Fördern des pharmazeutischen Fluids in ausschließlich eine Flussrichtung, also aus dem ersten Reservoir in das zweite Reservoir oder aus dem zweiten Reservoir in das erste Reservoir, zuzulassen, ist eine Ausgestaltungsform des Marknagels dadurch gekennzeichnet, dass das zweite Leitungsmittel ein zweites Rückschlagventil aufweist.

Eine Ausgestaltungsform des Marknagels ist dadurch gekennzeichnet, dass das zweite Rückschlagventil in Richtung der Außenfläche fluidundurchlässig und in Richtung des zweiten Anschlusses fluiddurchlässig ausgestaltet ist. Dadurch erlaubt der Marknagel ein Fördern des pharmazeutischen Fluids ausschließlich aus dem ersten Reservoir in das zweite Reservoir. Ein Vorteil dieser Ausgestaltungsform ist, dass das nur noch nicht mit Knochengewebe in Kontakt gekommenes pharmazeutisches Fluid durch das zumindest partiell im Marknagel verlaufende erste Leitungsmittel förderbar ist und eine Kontamination und/oder eine Verstopfung des ersten Leitungsmittel mit Knochengewebe, welches sich nach dem Durchspülen des Knochenkanals im pharmazeutischen Fluid ansammeln kann, ausgeschlossen wird.

Das zweite Rückschlagventil kann unterschiedliche Bauformen aufweisen. In einer Ausgestaltungsform umfasst das zweite Rückschlagventil eine Rückschlagklappe, welche ein Fördern des pharmazeutischen Fluids durch das zweite Leitungsmittel nur in eine Flussrichtung erlaubt und in die entgegengesetzte Flussrichtung das zweite Leitungsmittel fluidleitend verschließt.

Ein Ausgestaltungsform des Marknagels ist dadurch gekennzeichnet, dass das zweite Rückschlagventil ein zweites Rückstellelement aufweist, welche das zweite Rückschlagventil in eine Flussrichtung fluidleitend verschließt und in die entgegengesetzte Flussrichtung ein Fördern des pharmazeutischen Fluids durch das zweite Rückschlagventil erlaubt. Aufgrund der einfachen Bauweise und der sicheren Verwendbarkeit umfasst das zweite Rückstellelement bevorzugt eine Feder, insbesondere eine Spiralfeder, beispielsweise aus einem Kunststoff oder aus einem Metall, oder besteht das zweite Rückstellelement aus einer Feder, insbesondere einer Spiralfeder, beispielsweise aus einem Kunststoff oder aus einem Metall.

In einer Ausgestaltungsform ist das zweite Rückschlagventil als Kugelrückschlagventil ausgebildet. Aufgrund der hohen strukturellen Robustheit ist in einer weiteren, bevorzugten, Ausgestaltungsform das zweite Rückschlagventil als Tellerrückschlagventil ausgebildet.

Eine Ausgestaltungsform des Marknagels ist dadurch gekennzeichnet, dass der Marknagel mindestens eine Bohrung aufweist, um den Marknagel innerhalb eines Knochenkanals zu fixieren. Dazu kann jeweils ein Befestigungsmittel, wie beispielsweise eine Schraube, durch die mindestens eine Bohrung sowie durch das den Marknagel umgebendes Knochengewebe getrieben werden, wodurch eine Verschiebung und/oder Verdrehung des Marknagels innerhalb des Knochenkanals unterbunden wird. Bevorzugt weist der Marknagel die zumindest eine Bohrung am ersten Ende oder am zweiten Ende des Marknagels auf. Weiter bevorzugt weist der Marknagel mindestens eine Bohrung am ersten Ende und mindestens eine Bohrung am zweiten Ende auf.

Eine Ausgestaltungsform des Marknagels ist dadurch gekennzeichnet, dass der Marknagel mindestens einen Dichtungsring aufweist. Der mindestens eine Dichtungsring umschließt die Außenfläche des Marknagels fluidundurchlässig und wirkt nach einer Implantation des Marknagels derart mit dem den Marknagel umgebenden Knochengewebe zusammen, dass ein pharmazeutisches Fluid, insbesondere ein flüssiges pharmazeutisches Fluid, nicht zwischen Dichtungsring und Knochengewebe hindurch förderbar ist. Beispiele für Dichtungsringe umfassen Schaumringe aus einem geschäumten Kunststoff oder massive, nicht geschäumte elastische Kunststoffringe, insbesondere Dichtungsringe aus Elastomeren. In einer Ausgestaltungsform weist der Marknagel mindestens einen Dichtungsring am ersten Ende auf. In einer weiteren Ausgestaltungsform weist der Marknagel mindestens einen Dichtungsring am zweiten Ende auf. In einer weiteren, bevorzugten, Ausgestaltungsform weist der Marknagel mindestens einen Dichtungsring am ersten Ende und mindestens einen Dichtungsring am zweiten Ende auf, derart. Der mindestens eine Dichtungsring am ersten Ende und der mindestens eine Dichtungsring am zweiten Ende umfassen mindestens das an der Außenfläche verlaufende zweite Leitungsmittel derart, dass das pharmazeutische Fluid kontrolliert von dem einen Reservoir in das andere Reservoir förderbar ist, verhindern aber gleichzeitig ein ungewolltes Verteilen des pharmazeutischen Fluids in Richtung und über die beiden axialen Enden des Marknagels hinaus. Dies unterbindet ein ungewolltes Anreicherung des im pharmazeutischen Fluids enthaltenen Wirkstoffs im Patienten.

Ein weiterer Gegenstand, welcher nicht Teil der Erfindung ist, betrifft ein Verfahren zur Behandlung von Knochenfrakturen und zur Applikation eines pharmazeutischen Fluids in den Bereich des Knochenkanals mittels eines Marknagels, insbesondere eines Marknagels nach einer der vorhergehenden Ausgestaltungsformen. Das Verfahren umfasst zumindest die folgenden Schritte:
a) Implantieren des Marknagels in den Knochenkanal;
b) Fluidleitendes Verbinden des ersten Leitungsmittels mit dem ersten Reservoir und des zweiten Leitungsmittels mit dem zweiten Reservoir;
c1) zumindest partielles Fördern des pharmazeutischen Fluids aus dem ersten Reservoir über das erste Leitungsmittel, die mindestens eine Durchführung, die Außenfläche und das zweite Leitungsmittel in das zweite Reservoir; und/oder
c2) zumindest partielles Fördern des pharmazeutischen Fluids aus dem zweiten Reservoir über das zweite Leitungsmittel, die Außenfläche, die mindestens eine Durchführung und das erste Leitungsmittel in das erste Reservoir.

In einer Ausgestaltung des Verfahrens werden sowohl die Schritte c1) als auch c2) durchgeführt, so dass das pharmazeutische zumindest partiell abwechselnd zwischen dem ersten Reservoir und dem zweiten Reservoir verbracht wird.

In einer weiteren, bevorzugten, Ausgestaltungsform des Verfahrens wird entweder Schritt c1) oder Schritt c2) durchgeführt, so dass das pharmazeutische Fluid nur einmal in eine Flussrichtung durch den Marknagel gefördert wird. Dies verhindert eine Kontamination und/oder eine Verstopfung des Marknagels durch Knochengewebe, insbesondere durch Knochenmark.

Falls das Verfahren nur den Schritt c1) oder den Schritt c2) umfasst, also das pharmazeutische nur einmal durch den Marknagel gefördert wird, kann das das pharmazeutische Fluid aufnehmende erste Reservoir oder zweite Reservoir auf unterschiedliche Weise in Schritt b) mit dem ersten Leitungsmittel beziehungsweise mit dem zweiten Leitungsmittel fluidleitend verbunden werden. In einer Ausgestaltungsform wird das das pharmazeutische Fluid aufnehmende Reservoir direkt physisch mit dem entsprechenden Leitungsmittel, beispielsweise über einen Schlauch, verbunden. In einer weiteren Ausgestaltungsform wird das das pharmazeutische Fluid aufnehmende Reservoir, beispielsweise eine Schale oder ein Gefäß, nicht direkt physisch mit dem entsprechenden Leitungsmittel verbunden, sondern das entsprechende Reservoir fängt das aus dem entsprechenden Leitungsmittel, beispielsweise als Tropfen, austretende pharmazeutische Fluid lediglich auf.

In einer bevorzugten Ausgestaltungsform des Verfahrens umfasst das Verfahren den Schritt c1) und nicht den Schritt c2). Dadurch wird das erste Leitungsmittel, welches sich in einer bevorzugten Ausgestaltungsform im Vergleich zum zweiten Leitungsmittel über eine längere Strecke innerhalb des Marknagel erstreckt, vor Kontamination und Verstopfung geschützt.

Die Applikation des pharmazeutischen Fluids kann auf unterschiedliche Weise vonstattengehen. In einer Ausgestaltungsform wird das pharmazeutische Fluid über den Marknagel in den Knochenkanal appliziert, wo es über eine Zeitspanne von Minuten bis Tagen, beispielsweise bis zu 10 Tagen, im Bereich des Knochenkanals verbleibt. In einer weiteren Ausgestaltungsform wird das pharmazeutische Fluid ein oder mehrmals kontinuierlich, ohne zeitlichen Verzug, durch den Marknagel gefördert, so dass der Knochenkanal ein oder mehrmals mit dem pharmazeutischen Fluid durchspült wird.

Die für den Marknagel offenbarten Merkmale sind auch für das Verfahren offenbart und umgekehrt.

### Beispiele

Die Erfindung wird im Folgenden durch Beispiele weiter beispielhaft illustriert. Die Erfindung ist nicht auf die Beispiele beschränkt.

### Figuren

Es zeigen
- Fig. 1: einen schematischen Querschnitt eines Marknagels,
- Fig. 2: eine schematischen Außenansicht des Marknagels,
- Fig. 3: einen schematischen Querschnitt des Marknagels umfassend ein erstes Rückschlagventil in einer geschlossenen Position,
- Fig. 4: einen schematischen Querschnitt des Marknagels umfassend das erste Rückschlagventil in einer offenen Position,
- Fig. 5: einen schematischen Querschnitt des Marknagels umfassend ein zweites Rückschlagventil in einer geschlossenen Position,
- Fig. 6: einen schematischen Querschnitt des Marknagels umfassend das zweite Rückschlagventil in einer offenen Position, und
- Fig. 7: ein Verfahren zum Behandeln eines Patienten mittels eines Marknagels.

### Beschreibung der Figuren

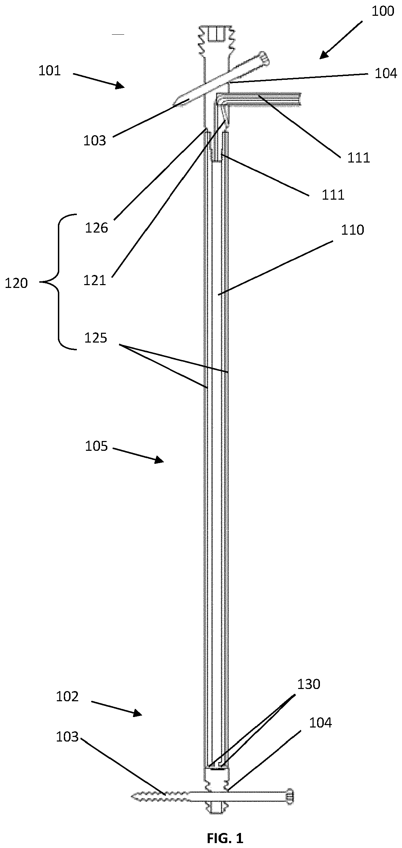

**Figur 1** zeigt einen schematischen Querschnitt eines Marknagels 100. Der Marknagel 100 weist ein axial verlaufendes erstes Leitungsmittel 110 auf. Das erste Leitungsmittel 110 verläuft von einem ersten Ende 101 axial bis zu einem zweiten Ende 102 des Marknagels 100. Das erste Leitungsmittel 110 weist am ersten Ende 101 einen ersten Anschluss 111 auf, über den der Marknagel 100, insbesondere das erste Leitungsmittel 110, mit einem ersten Reservoir für ein pharmazeutisches Fluid fluidleitend verbindbar ist. Das erste Leitungsmittel 110 ist partiell als axial verlaufender Kanal innerhalb des Marknagels 100 ausgebildet, durch den ein pharmazeutisches Fluid, insbesondere ein flüssiges pharmazeutisches Fluid, förderbar ist. Am zweiten Ende 102 weist der Marknagel 100 Durchführungen 130 auf, welche das erste Leitungsmittel 110 mit einem zweiten Leitungsmittel 120 und über das zweite Leitungsmittel 120 auch mit einer Außenfläche 105 des Marknagels 100 fluidleitend verbinden. Der gezeigte Marknagel 100 weist vier Durchführungen (wobei eine Durchführung 130 aus der Zeichenebene heraus, und eine weitere Durchführung 130 hinter die Zeichenebene gerichtet, und somit nicht sichtbar, ist) auf, welche jeweils mit einem Winkel von 90° radial versetzt zueinander am Marknagel 100 angeordnet sind. In weiteren, nicht gezeigten, Ausgestaltungsformen des Marknagels 100 kann sowohl die Anzahl als auch die Anordnung der Durchführungen 130 variieren.

Das zweite Leitungsmittel 120 umfasst in der gezeigten Ausgestaltungsform vier axial in der Außenfläche 105 verlaufende Nuten 125 (wobei wiederum, analog zu den Durchführungen 130, eine der axialen Nuten 125 vor der Zeichenebene, und eine weitere Nut 125 hinter der Zeichenebene verläuft), welche sich unmittelbar an die vier Durchführungen 130 anschließen, eine radial umlaufende Vertiefung 126 sowie einen kanalartigen zweiten Anschluss 121 am ersten Ende 101 des Marknagels 100, wobei die kanalartige Vertiefung 126 den zweiten Anschluss 121 und die Nuten 125 fluidleitend verbindet.

In der gezeigten Ausgestaltungsform des Marknagels 100 sind der erste Anschluss 111 und der zweite Anschluss 121 einstückig ausgebildet, derart, dass ein erstes Reservoir und ein zweites Reservoir mit einem Doppelschlauchsystem an den Marknagel 100 anschließbar ist. In weiteren, nicht gezeigten, Ausgestaltungsform können der erste Anschluss 111 und der zweite Anschluss 121 separat voneinander ausgestaltet sein, so dass ein erstes Reservoir und ein zweites Reservoir mit jeweils einem separaten Anschlussmittel, wie beispielsweise einem Schlauch, an den ersten Anschluss 111 und den zweiten Anschluss 121 anschließbar ist. In der gezeigten Ausgestaltungsform ist das erste Ende 101 mittels einer Schraubverbindung 107 am Rest des Marknagels 100 befestigt. In weiteren, nicht gezeigten, Ausgestaltungsformen ist das erste Ende 101 und der Rest des Marknagels 100 einteilig ausgestaltet.

Der Marknagel 100 erlaubt ein Fördern eines pharmazeutischen Fluid in zwei Flussrichtungen. In einer ersten Ausgestaltungsform kann das pharmazeutische Fluid über den ersten Anschluss 111 in das erste Leitungsmittels 110 in den Marknagel 100 eingeleitet und Richtung des zweiten Ende 102 durch das erste Leitungsmittel 110 gefördert werden, wobei es bei Erreichen des zweiten Endes 102 durch die Durchführungen 130 aus dem Marknagel 100 in die Nuten 125 geleitet wird. Die Nuten 125 leiten das pharmazeutische Fluid wieder zurück in Richtung des ersten Endes 101, wo es durch die radial umlaufende Vertiefung 126 eingesammelt und mittels des zweiten Anschlusses 121 in ein zweites Reservoir geleitet wird. In einer weiteren Ausgestaltungsform kann das pharmazeutische Fluid aus einem zweiten Reservoir über den zweiten Anschluss 121 in das zweite Leitungsmittel 120 in den Marknagel 100 eingeleitet und Richtung des zweiten Endes 102 gefördert werden, wobei es zuerst durch die radial umlaufende Vertiefung 126 und im Folgenden über die Nuten 125 geführt wird. Bei Erreichen des zweiten Endes 102 wird das pharmazeutische Fluid aus den Nuten 125 durch die Durchführungen 130 in das erste Leitungsmittel 110 eingebracht. Das erste Leitungsmittel 110 leitet das pharmazeutische Fluid wieder zurück Richtung des ersten Endes 101, wobei es bei Erreichen des ersten Endes 101 mittels des ersten Anschlusses 111 aus dem Marknagel 100 in ein erstes Reservoir überführt wird.

In beiden Ausgestaltungsformen steht das pharmazeutische Fluid über die Nuten 125 mit der Außenfläche 105 des Marknagels 100 fluidleitend in Verbindung, was eine Applikation des im pharmazeutischen Fluid enthaltenen Wirkstoffs an das umgebende Knochengewebe des implantierten Marknagels 100 erlaubt.

Am ersten Ende 101 und am zweiten Ende 102 sind mit jeweils einem Befestigungsmittel 103 in Form einer Schraube versehene Bohrungen 104 angebracht. Die Befestigungsmittel 103 dienen dazu, den Marknagel 100 innerhalb eines Knochenkanals räumlich zu fixieren.

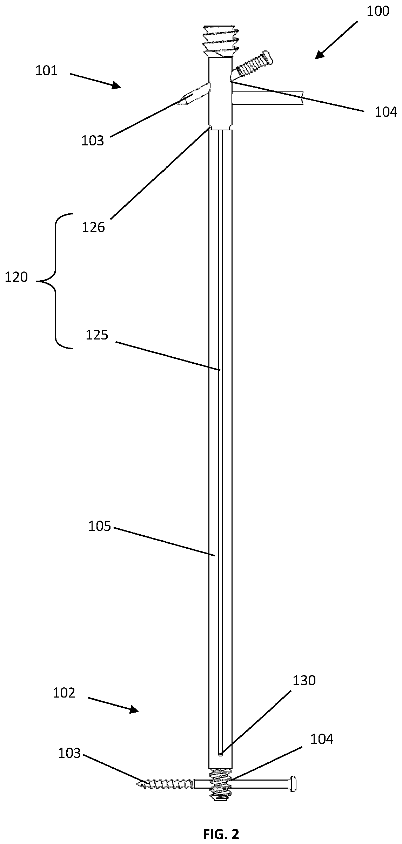

**Figur 2** zeigt eine schematische Außenansicht des Marknagels 100 aus Figur 1. Am zweiten Ende 102 mündet in der Draufsicht eine der Durchführungen 130 aus dem Inneren des Marknagels 100 in eine der Nuten 125. Die entsprechende Nut 125 verläuft vom zweiten Ende 102 axial bis zum ersten Ende 101 in der Außenfläche 105 des Marknagels 100, wo sie fluidleitend mit der radial umlaufenden Vertiefung 126 verbunden ist. Durch die fluidleitende Verbindung der Nuten 125 mit der Außenfläche 105 steht somit ein Großteil der Gesamtlänge des Marknagels 100 für eine großflächige Applikation eines im pharmazeutischen Fluids enthaltenen Wirkstoffs an die Umgebung des Marknagels 100 zur Verfügung.

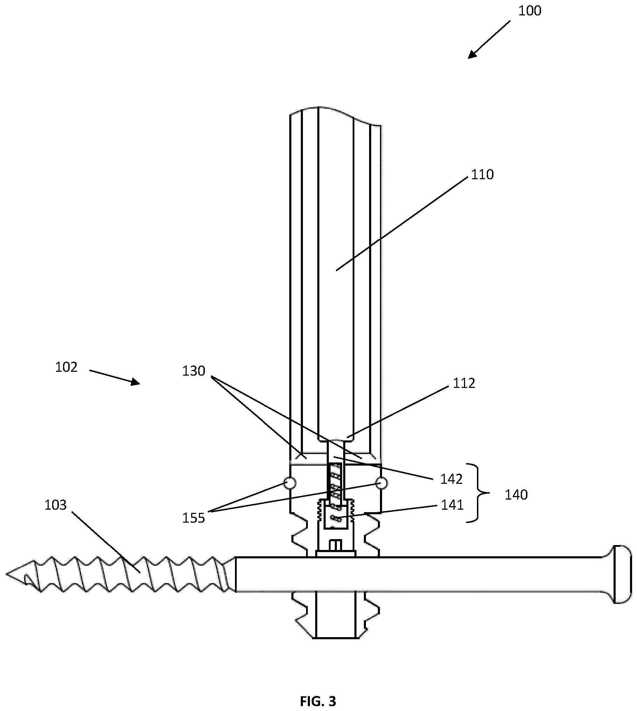

**Figur 3** zeigt einen Ausschnitt eines schematischen Querschnitts des Marknagels 100 aus den Figuren 1 und 2, wobei das erste Leitungsmittel 110 zusätzlich ein erstes Rückstellelement 140 aufweist. Das erste Rückschlagventil befindet sich an einem dem zweiten Ende 102 des Marknagels 100 zugewandten Ende 112 des ersten Leitungsmittels 110, direkt am Übergang zu den Durchführungen 130 und befindet sich in einer geschlossenen Position. Dazu spannt ein erstes Rückstellelement 141 in Form einer Feder einen ersten Kolben 142 derart vor, dass dieser den Übergang von erstem Leitungsmittel 110 und den Durchführungen 130 fluidleitend verschließt. In der geschlossenen Position ist ein Fördern eines pharmazeutischen Fluids von außerhalb des Marknagels 100 durch die Durchführungen 130 in das erste Leitungsmittel 110 nicht möglich, so dass das Risiko einer Kontamination und/oder einer Verstopfung des ersten Leitungsmittels 110 verhindert wird.

In der gezeigten Ausgestaltungsform weist der Marknagel 100 am zweiten Ende 102 einen zweiten Dichtungsring 155 auf, welcher den Marknagel 100 radial umfasst. Der zweite Dichtungsring 155 wirkt nach einer Implantation des Marknagels 100 derart mit dem den Marknagel 100 umgebenden Knochengewebe zusammen, dass ein durch den Marknagel 100 gefördertes pharmazeutisches Fluid, welches aus den Durchführungen 130 austritt, in Richtung des ersten Endes 101 und nicht in Richtung des Befestigungsmittels 103 des zweiten Endes 102 geleitet wird. Dadurch wird eine unkontrollierte Anreicherung des im pharmazeutischen Fluid enthalten Wirkstoff im Körper des Patienten verhindert. In einer weiteren, nicht gezeigten, Ausgestaltungsform weist der Marknagel 100 zwar den zweiten Dichtungsring 155, aber nicht das erste Rückschlagventil 110 auf.

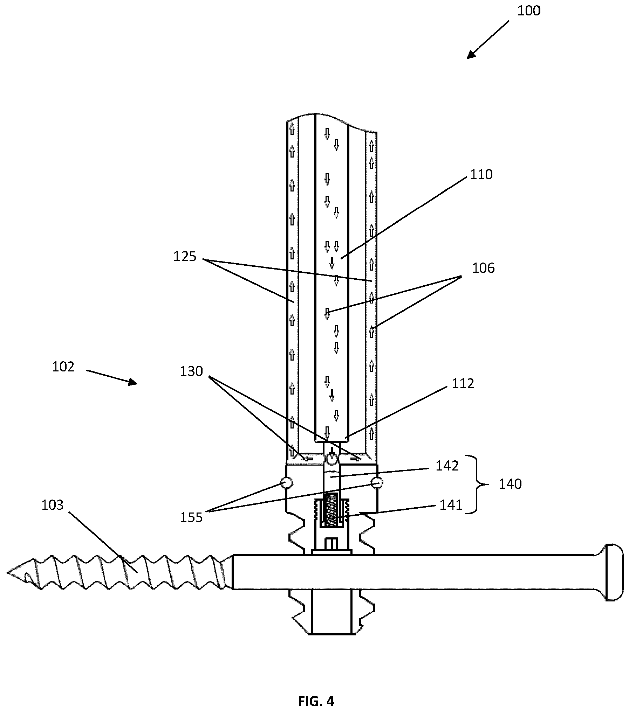

**Figur 4** zeigt einen Ausschnitt eines schematischen Querschnitts des Marknagels 100 aus den Figuren 1, 2 und 3, wobei das zusätzliche erste Rückschlagventil 140 aus Figur 3 in einer offenen Position dargestellt ist. Figur 4 zeigt ein Fördern eines pharmazeutischen Fluids durch das erste Leitungsmittel 110 in Richtung der Durchführungen 130 und entlang der Nuten 125 wieder zurück zum ersten Ende 101 des Marknagels 100, wie anhand der Pfeile 106 illustriert. Ausgelöst durch einen Förderdruck des pharmazeutischen Fluid durch das erste Leitungsmittel 110 in Richtung des zweiten Endes 102, befindet sich das erste Rückschlagventil 140 in der offenen Position, indem das pharmazeutische Fluid den ersten Kolben 142 des ersten Rückschlagventils 140 entgegen der Spannkraft des ersten Rückstellelements 141 in Richtung der Bohrung 104 am zweiten Ende 102 verschiebt. Das erste Rückschlagventil erlaubt somit ein Fördern eines pharmazeutischen Fluids nur entlang der durch die Pfeile 106 illustrierten Flussrichtung, wodurch das Risiko einer Kontamination und/oder einer Verstopfung des ersten Leitungsmittels 110 verringert wird.

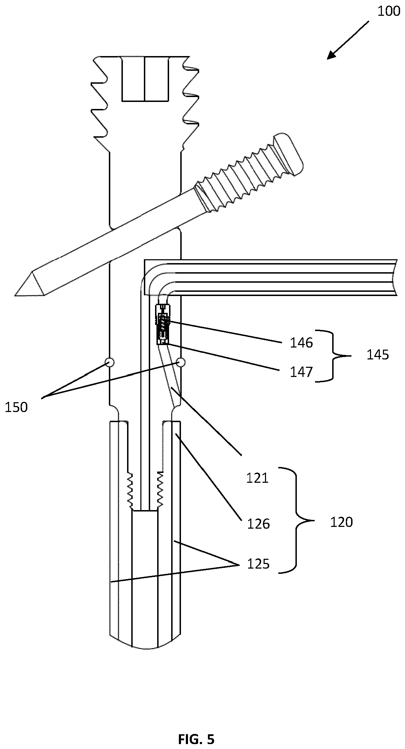

**Figur 5** zeigt einen Ausschnitt eines schematischen Querschnitts des Marknagels 100 aus den vorhergehenden Figuren, wobei das zweite Leitungsmittel 120, insbesondere der zweite Anschluss 121, zusätzlich ein zweites Rückschlagventil 145 aufweist. Figur 5 zeigt das zweite Rückschlagventil 145 in einer geschlossenen Position. Dazu spannt ein zweites Rückstellelement 146 in Form einer Feder einen zweiten Kolben 147 derart vor, dass dieser das zweite Leitungsmittel 120, insbesondere den zweiten Anschluss 121 in Flussrichtung der radialen Vertiefung fluidleitend verschließt. In der geschlossenen Position des zweiten Rückschlagventils 145 ist ein Fördern eines pharmazeutischen Fluids durch den zweiten Anschluss 121 in Richtung des zweiten Ende 102 des Marknagels 100 nicht möglich.

In einer ersten Ausgestaltungsform weist der Marknagel 100 nur das zweite Rückschlagventil 145 auf. In einer weiteren, bevorzugten, Ausgestaltungsform weist der Marknagel 100 das erste Rückschlagventil 140 gemäß den Figuren 3 und 4 und das zweite Rückschlagventil 145 auf. Das zweite Rückschlagventil 145 ist im kanalartigen zweiten Anschluss 121, insbesondere an einem der radialen Vertiefung 126 zugewandten Ende des zweiten Anschlusses 121, angeordnet.

In der gezeigten Ausgestaltungsform weist der Marknagel 100 am ersten Ende 101 einen ersten Dichtungsring 150 auf, welcher den Marknagel 100 radial umfasst. Der erste Dichtungsring 150 wirkt nach einer Implantation des Marknagels 100 derart mit dem den Marknagel 100 umgebenden Knochengewebe zusammen, dass ein durch den Marknagel 100 gefördertes pharmazeutisches Fluid, welche durch die Nuten 125 in Richtung des ersten Endes 101 gefördert wird in den zweiten Anschluss 121 und nicht in Richtung des Befestigungsmittels 103 am ersten Ende 101 abgeleitet wird. Dadurch wird eine unkontrollierte Anreicherung des im pharmazeutischen Fluid enthalten Wirkstoff im Körper des Patienten verhindert. In einer weiteren, nicht gezeigten, Ausgestaltungsform weist der Marknagel 100 zwar den zweiten Dichtungsring 155, aber nicht das erste Rückschlagventil 110 auf. In einer weiteren Ausgestaltungsform weist der Marknagel sowohl den ersten Dichtungsring 150 als auch den zweiten Dichtungsring 155 auf. Dadurch ist ein kontrolliertes Fördern eines pharmazeutischen Fluids aus einem Reservoir in ein anderes Reservoir, bevorzugt aus dem ersten Reservoir in das zweite Reservoir, möglich, ohne dass es zu einer ungewollten Anreicherung des im pharmazeutischen Fluids enthaltenen Wirkstoffs kommt.

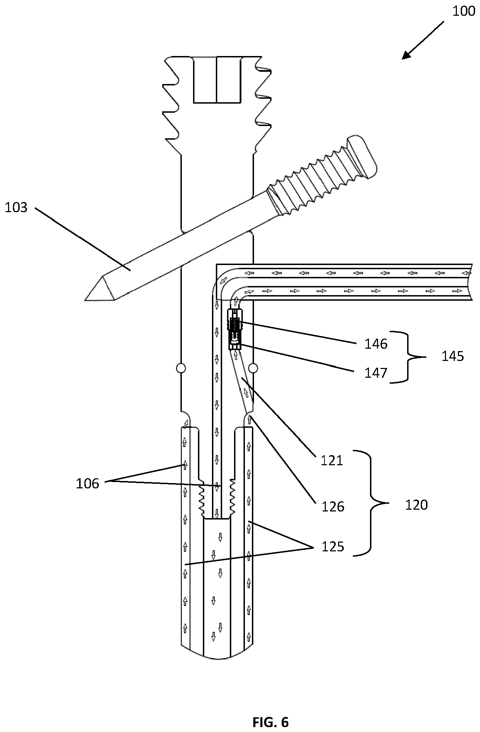

**Figur 6** zeigt den Ausschnitt eines schematischen Querschnitts des Marknagels 100 aus den vorhergehenden Figuren, wobei das zusätzliche zweite Rückschlagventil 145 aus Figur 5 in einer offenen Position dargestellt ist. Figur 6 zeigt ein Fördern eines pharmazeutischen Fluids durch das erste Leitungsmittel 110 in Richtung des zweiten Endes 102 und durch das zweite Leitungsmittel 120 wieder zurück in Richtung des ersten Ende 101 des Marknagels, wie anhand der Pfeile 106 illustriert. Ausgelöst durch einen Förderdruck, welcher durch das Fördern des pharmazeutischen Fluids durch den Marknagel 100 hervorgerufen wird, befindet sich das zweite Rückschlagventil 145 in der offenen Position, indem das pharmazeutische Fluid den zweiten Kolben 147 des zweiten Rückschlagventils 145 entgegen der Spannkraft des zweiten Rückstellelements 141 in Richtung der Bohrung 104 am ersten Ende 101 verschiebt. Das zweite Rückschlagventil erlaubt somit ein Fördern eines pharmazeutischen Fluids nur der entlang der Pfeile 106 illustrierten Flussrichtung, wodurch das Risiko einer Kontamination und/oder einer Verstopfung, insbesondere des ersten Leitungsmittels 110, verringert wird.

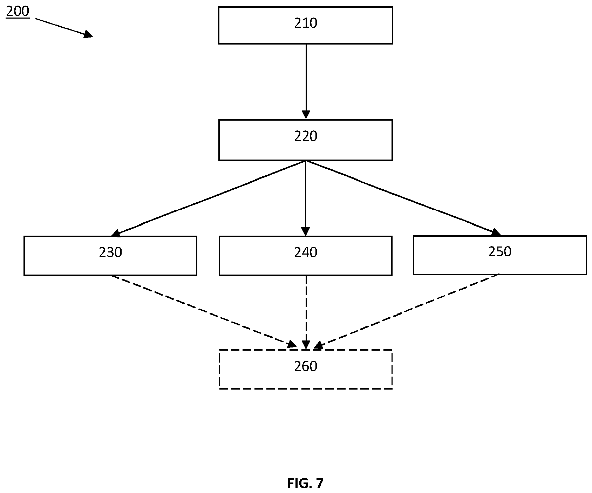

**Figur 7** zeigt ein Flussdiagram eines Verfahren 200 zur Behandlung von Knochenfrakturen und zur Applikation eines pharmazeutischen Fluids in den Bereich des Knochenkanals mittels eines Marknagels, umfassend die Schritte 210, 220, 230 und/oder 240 sowie einen optionalen Schritt 250. In Schritt 210 wird der Marknagel in den Knochenkanal implantiert und in Schritt 220 ein erstes Leitungsmittel des Marknagels mit einem ersten Reservoir für pharmazeutische Fluids sowie ein zweites Leitungsmittel des Marknagels mit einem zweiten Reservoir für pharmazeutische Fluids fluidleitend, beispielsweise über einen oder mehrere Schläuche, verbunden. Im Folgenden kann das Verfahren 200 auf drei unterschiedliche Arten durchgeführt werden. In einer ersten Ausgestaltungsform des Verfahrens 200 wird ein pharmazeutisches Fluid in Schritt 230 vom ersten Reservoir zumindest partiell in das zweite Reservoir, beispielsweise über pumpen, gefördert. Ein Fördern des pharmazeutischen Fluids vom zweiten Reservoir in das erste Reservoir findet nicht statt. In einer zweiten Ausgestaltungsform des Verfahrens 200 wird ein pharmazeutisches Fluid in Schritt 240 vom zweiten Reservoir zumindest partiell in das erste Reservoir, beispielsweise über pumpen, gefördert. Ein Fördern des pharmazeutischen Fluids vom ersten Reservoir in das zweite Reservoir findet nicht statt. In der dritten Ausgestaltungsform des Verfahrens 200 wird in einem Schritt 250 sowohl ein zumindest partielles Fördern eines pharmazeutischen Fluid vom ersten Reservoir in das zweite Reservoir als auch ein zumindest partielles Fördern vom zweiten Reservoir in das erste Reservoir durchgeführt. Dabei können das erste Reservoir und das zweite Reservoir jeweils das gleiche pharmazeutische Fluid oder jeweils ein unterschiedliches pharmazeutisches Fluid vor dem ersten Fördern lagern oder jeweils nur das erste Reservoir oder nur das zweite Reservoir lagern vor dem ersten Fördern ein pharmazeutisches Fluid. Nach Beendigung der Schritte 230, 240 oder 250 wird in einem optionalen Schritt 260 das erste Reservoir vom ersten Leitungsmittel und das zweite Reservoir vom zweiten Leitungsmittel fluidleitend getrennt.

Die in den Ansprüchen, der Beschreibung und in den Figuren offenbarten Merkmale können für verschiedene Ausgestaltungsformen der beanspruchten Erfindung sowohl getrennt als auch in beliebiger Kombination miteinander wesentlich sein. Die für den Marknagel offenbarten Merkmale sind auch für das Verfahren offenbart und umgekehrt.

### Bezugszeichen

- 100: Marknagel
- 101: erstes Ende
- 102: zweites Ende
- 103: Befestigungsmittel
- 104: Bohrung
- 105: Außenfläche
- 106: Pfeile
- 107: Schraubverbindung
- 110: erstes Leitungsmittel
- 111: erster Anschluss
- 120: zweites Leitungsmittel
- 121: zweiter Anschluss
- 125: Nut
- 126: radiale Vertiefung
- 130: Durchführung
- 140: erstes Rückschlagventil
- 141: erstes Rückstellelement
- 142: erster Kolben
- 145: zweites Rückschlagventil
- 146: zweites Rückstellelement
- 147: zweiter Kolben
- 150: erster Dichtungsring
- 155: zweiter Dichtungsring
- 200: Verfahren zur Behandlung von Knochenfrakturen
- 210: Implantation
- 220: fluidleitendes Verbinden
- 230: Fördern von erstem Reservoir in zweites Reservoir
- 240: Fördern von zweitem Reservoir in erstes Reservoir
- 250: Fördern von erstem Reservoir in zweites Reservoir und von zweitem Reservoir in erstes Reservoir
- 260: optionales fluidleitendes Trennen

## Patentansprüche

1. Marknagel (100) zur Applikation eines pharmazeutischen Fluids, umfassend
ein axial im Marknagel (100) verlaufendes fluidleitendes erstes Leitungsmittel (110),
welches mit einem ersten Reservoir für das pharmazeutische Fluid verbindbar ist und mindestens eine Durchführung (130), welche das erste Leitungsmittel (110) fluidleitend mit einer Außenfläche (105) des Marknagels (100) verbindet,
**dadurch gekennzeichnet, dass**
ein fluidleitendes zweites Leitungsmittel (120) die Außenfläche (105) derart mit einem zweiten Reservoir verbindet, um das pharmazeutische Fluid zwischen dem ersten Reservoir und dem zweiten Reservoir zu fördern.

2. Marknagel (100) nach Anspruch 1, **dadurch gekennzeichnet, dass** das erste Leitungsmittel (110) einen ersten Anschluss (111) zur fluidleitenden Verbindung mit dem ersten Reservoir und das zweite Leitungsmittel (120) einen zweiten Anschluss (121) zur fluidleitenden Verbindung mit dem zweiten Reservoir umfasst, wobei der erste Anschluss (111) und der zweite Anschluss (121) an einem ersten Ende (101) des Marknagels (100) angeordnet sind.

3. Marknagel (100) nach Anspruch 2, **dadurch gekennzeichnet, dass** die mindestens eine Durchführung (130) an einem dem ersten Ende (101) entgegengesetzten zweitem Ende (102) des Marknagels (100) angeordnet ist.

4. Marknagel (100) nach einem der vorhergehenden Ansprüche, **dadurch gekennzeichnet, dass** das zweite Leitungsmittel (120) zumindest partiell als mindestens eine sich axial erstreckende Nut (125) in der Außenfläche (105) ausgebildet ist.

5. Marknagel (100) nach Anspruch 4, **dadurch gekennzeichnet, dass** die mindestens eine sich axial erstreckende Nut (125) fluidleitend mit der mindestens einen Durchführung (130) verbunden ist.

6. Marknagel (100) nach Anspruch 4 oder 5, **dadurch gekennzeichnet, dass** das zweite Leitungsmittels (120) als zwei bis sechs, insbesondere als vier, sich axial erstreckende Nuten (125) in der Außenfläche (105) ausgebildet ist und der Marknagel (100) die gleiche Anzahl an Durchführungen (130) aufweist, wobei jeweils eine Nut (125) mit jeweils einer Durchführungen (130) fluidleitend miteinander verbunden ist.

7. Marknagel (100) nach einem der vorhergehenden Ansprüche, **dadurch gekennzeichnet, dass** das erste Leitungsmittel (110) ein erstes Rückschlagventil (140) aufweist.

8. Marknagel (100) nach Anspruch 7, **dadurch gekennzeichnet, dass** das erste Rückschlagventil (140) in Richtung der mindestens einen Durchführung (130) fluiddurchlässig ausgestaltet ist.

9. Marknagel (100) nach Anspruch 7 oder 8, **dadurch gekennzeichnet, dass** das erste Rückschlagventil (140) ein erstes Rückstellelement (141), insbesondere eine erste Feder, aufweist.

10. Marknagel (100) nach einem der vorhergehenden Ansprüche, **dadurch gekennzeichnet, dass** das zweite Leitungsmittel (120) ein zweites Rückschlagventil (145) aufweist.

11. Marknagel (100) nach Anspruch 10, **dadurch gekennzeichnet, dass** das zweite Rückschlagventil (145) in Richtung der Außenfläche (105) fluidundurchlässig ausgestaltet ist.

12. Marknagel (100) nach Anspruch 10 oder 11, **dadurch gekennzeichnet, dass** das zweite Rückschlagventil (145) ein zweites Rückstellelement (146), insbesondere eine zweite Feder, aufweist.

13. Marknagel (100) nach einem der vorhergehenden Ansprüche, **dadurch gekennzeichnet, dass** der Marknagel (100) mindestens eine Bohrung (104) aufweist, um den Marknagel (100) innerhalb eines Knochenkanals zu fixieren.

## Claims

1. An intramedullary nail (100) for applying a pharmaceutical fluid, comprising
a fluid-conducting first conduction means (110) which extends axially in the intramedullary nail (100) and can be connected to a first reservoir for the pharmaceutical fluid, and
at least one passage (130) which connects the first conduction means (110) to an outer surface (105) of the intramedullary nail (100) in a fluid-conducting manner,
**characterized in that**
a fluid-conducting second conduction means (120) connects the outer surface (105) to a second reservoir so as to convey the pharmaceutical fluid between the first reservoir and the second reservoir.

2. The intramedullary nail (100) according to claim 1, **characterized in that** the first conduction means (110) comprises a first connector (111) for fluid-conducting connection to the first reservoir and the second conduction means (120) comprises a second connector (121) for fluid-conducting connection to the second reservoir, the first connector (111) and the second connector (121) being arranged at a first end (101) of the intramedullary nail (100).

3. The intramedullary nail (100) according to claim 2, **characterized in that** the at least one passage (130) is arranged at a second end (102) of the intramedullary nail (100) opposite the first end (101).

4. The intramedullary nail (100) according to any of the preceding claims, **characterized in that** the second conduction means (120) is formed at least partly as at least one axially extending groove (125) in the outer surface (105).

5. The intramedullary nail (100) according to claim 4, **characterized in that** the at least one axially extending groove (125) is connected to the at least one passage (130) in a fluid-conducting manner.

6. The intramedullary nail (100) according to either claim 4 or claim 5, **characterized in that** the second conduction means (120) is formed as two to six, in particular as four, axially extending grooves (125) in the outer surface (105), and the intramedullary nail (100) comprises the same number of passages (130), each groove (125) being connected to a passage (130) in each case in a fluid-conducting manner.

7. The intramedullary nail (100) according to any of the preceding claims, **characterized in that** the first conduction means (110) comprises a first non-return valve (140).

8. The intramedullary nail (100) according to claim 7, **characterized in that** the first non-return valve (140) is fluid-permeable in the direction of the at least one passage (130).

9. The intramedullary nail (100) according to either claim 7 or claim 8, **characterized in that** the first non-return valve (140) comprises a first restoring element (141), in particular a first spring.

10. The intramedullary nail (100) according to any of the preceding claims, **characterized in that** the second conduction means (120) comprises a second non-return valve (145).

11. The intramedullary nail (100) according to claim 10, **characterized in that** the second non-return valve (145) is fluid-impermeable in the direction of the outer surface (105).

12. The intramedullary nail (100) according to either claim 10 or claim 11, **characterized in that** the second non-return valve (145) comprises a second restoring element (146), in particular a second spring.

13. The intramedullary nail (100) according to any of the preceding claims, **characterized in that** the intramedullary nail (100) comprises at least one hole (104) for fixing the intramedullary nail (100) within a bone canal.

## Revendications

1. Clou intramédullaire (100) permettant l'application d'un fluide pharmaceutique, comprenant
un premier moyen de conduite (110) conducteur de fluide s'étendant axialement dans le clou intramédullaire (100) et pouvant être relié à un premier réservoir pour le fluide pharmaceutique et
au moins un passage (130) qui relie le premier moyen de conduite (110) à une surface extérieure (105) du clou intramédullaire (100) de manière à conduire du fluide,
**caractérisé en ce que**
un second moyen de conduite (120) conducteur de fluide relie la surface extérieure (105) à un second réservoir de manière à transporter le fluide pharmaceutique entre le premier réservoir et le second réservoir.

2. Clou intramédullaire (100) selon la revendication 1, **caractérisé en ce que** le premier moyen de conduite (110) comprend un premier raccord (111) pour une liaison conductrice de fluide avec le premier réservoir et le second moyen de conduite (120) comprend un second raccord (121) pour une liaison conductrice de fluide avec le second réservoir, le premier raccord (111) et le second raccord (121) étant disposés à une première extrémité (101) du clou intramédullaire (100).

3. Clou intramédullaire (100) selon la revendication 2, **caractérisé en ce que** l'au moins un passage (130) est disposé à une seconde extrémité (102) du clou intramédullaire (100) opposée à la première extrémité (101).

4. Clou intramédullaire (100) selon l'une quelconque des revendications précédentes, **caractérisé en ce que** le second moyen de conduite (120) est réalisé au moins partiellement sous la forme d'au moins une rainure s'étendant axialement (125) dans la surface extérieure (105).

5. Clou intramédullaire (100) selon la revendication 4, **caractérisé en ce que** l'au moins une rainure s'étendant axialement (125) est reliée à l'au moins un passage (130) de manière à conduire du fluide.

6. Clou intramédullaire (100) selon la revendication 4 ou 5, **caractérisé en ce que** le second moyen de conduite (120) est réalisé sous la forme de deux à six, en particulier quatre, rainures s'étendant axialement (125) dans la surface extérieure (105) et le clou intramédullaire (100) présente le même nombre de passages (130), respectivement une rainure (125) étant reliée à respectivement un passage (130) de manière à conduire du fluide entre eux.

7. Clou intramédullaire (100) selon l'une quelconque des revendications précédentes, **caractérisé en ce que** le premier moyen de conduite (110) présente un premier clapet anti-retour (140).

8. Clou intramédullaire (100) selon la revendication 7, **caractérisé en ce que** le premier clapet anti-retour (140) est conçu de manière à être perméable aux fluides dans la direction de l'au moins un passage (130).

9. Clou intramédullaire (100) selon la revendication 7 ou 8, **caractérisé en ce que** le premier clapet anti-retour (140) présente un premier élément de rappel (141), en particulier un premier ressort.

10. Clou intramédullaire (100) selon l'une quelconque des revendications précédentes, **caractérisé en ce que** le second moyen de conduite (120) présente un second clapet anti-retour (145).

11. Clou intramédullaire (100) selon la revendication 10, **caractérisé en ce que** le second clapet anti-retour (145) est conçu de manière à être imperméable aux fluides dans la direction de la surface extérieure (105).

12. Clou intramédullaire (100) selon la revendication 10 ou 11, **caractérisé en ce que** le second clapet anti-retour (145) présente un second élément de rappel (146), en particulier un second ressort.

13. Clou intramédullaire (100) selon l'une quelconque des revendications précédentes, **caractérisé en ce que** le clou intramédullaire (100) présente au moins un alésage (104) pour fixer le clou intramédullaire (100) à l'intérieur d'un canal osseux.
